# EUROPEAN PATENT SPECIFICATION

(11) **EP 1 453 786 B1**
(45) Date of publication and mention of the grant of the patent: **20.01.2010**
(21) Application number: 02778837.1
(22) Date of filing: 13.11.2002
(51) Int. Cl.: C07C 69/732, C07C 67/02, C08K 5/134, C09K 15/08

(54) **METHOD OF PREPARING HYDROXYALKYL HINDERED PHENOLIC ANTIOXIDANTS**
VERFAHREN ZUR HERSTELLUNG STERISCH GEHINDERTER HYDROXYALKYL SUBSTITUIERTER PHENOL-ANTIOXIDANTIEN
PROCEDE DE PREPARER DES ANTIOXYDANTS PHENOLIQUES HYDROXY ALKYLÉS STÉRIQUEMENT ENCOMBRÉS

(30) Priority: 14.12.2001 US 14913
(43) Date of publication of application: 08.09.2004
(73) Proprietor: Chemtura Corporation, Middlebury, CT 06749 (US)
(72) Inventor: Baranski, John, R., Southington, CT 06489 (US); Rowland, Robert G., Woodbridge, Conneticut 06525 (US)
(74) Representative: Serravalle, Marco
(86) International application number: PCT/US2002/036337
(87) International publication number: WO 2003/051816

(56) References cited:
- EP-A- 0 861 847
- WO-A-94/22945
- US-A- 5 892 097
- DATABASE CA [Online] CHEMICAL ABSTRACTS SERVICE, COLUMBUS, OHIO, US; TANAKA, SHINYA ET AL: "Phosphorous acid esters, their preparation, and stabilizers containing them" retrieved from STN Database accession no. 119:117545 CA XP002233718 & JP 05 086084 A (SUMITOMO CHEMICAL CO, JAPAN) 6 April 1993 (1993-04-06)
- DATABASE CA [Online] CHEMICAL ABSTRACTS SERVICE, COLUMBUS, OHIO, US; ALLEN, DAVID W. ET AL: "Characterization of electron beam generated transformation products of Irganox 1010 by particle beam liquid chromatography-mass spectrometry with online diode array detection" retrieved from STN Database accession no. 121:254142 CA XP002233719 & JOURNAL OF CHROMATOGRAPHY, A (1994), 679(2), 285-97 ,

## Description

### Background of the Invention

### 1. Field of the Invention

The present invention is directed to a method of preparing hindered phenolic antioxidants for lubrication oils or other organic media.

### 2. Description of Related Art

Hydroxyalkyl hindered phenolic antioxidants are used as stabilizers for lubrication oils or other organic media. They are generally the reaction product between a starting ester and an alcohol with one or more hydroxyl groups to produce the following mono- ester product species: 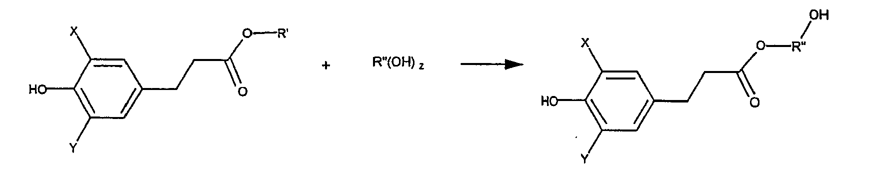 wherein R' and R" are hydrocarbon moieties.

Typically, the phenolic antioxidants are prepared using transesterification catalysts such as para-toluene sulfonic acid which need to be washed out of the final product. However, this method requires numerous reaction steps and generates large amounts of waste. Other transesterification catalysts such as dibutyl tin diacetate or titanium isopropoxide are used as well but the problem remained in substantially removing traces of the catalyst once the reaction was terminated. If the amount of residual metal in the final product is too high, ultimate product stability is compromised, and costly, time consuming efforts must be taken to remove or reduce the amount of residual metal to a satisfactory level.

U.S. Patent No. 5,892,097 to Ross et al. which issued on April 6, 1999, teaches a method of making esters of substituted hydroxyhydrocinnamic acid useful as antioxidants by the transesterification of the corresponding methyl or ethyl ester with a higher aliphatic alcohol using small amounts of a tin catalyst, between 10 and 250 ppm based on an amount of the starting ester. The low catalyst level purportedly eliminates the need for post reaction separation of catalyst from the final ester product.

However, although the levels of tin are desirably low in Ross et al., during work up of a reaction between the phenolic ester and the alkanol or polyol, any live catalyst remaining in the reaction mixture would generate further reaction as illustrated below. 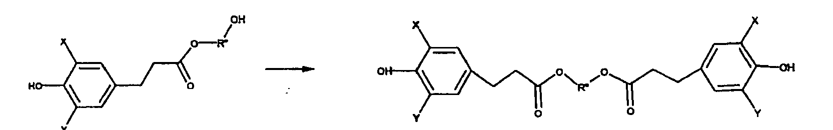 Reactions leading to the undesired bis- product species where the desired hydroxyl terminated mono- product species has further reacted are difficult to predict and control. Although the reaction may have proceeded to a majority of the mono- product species, during vacuum stripping of the solvents and/or alcohol, the remaining live catalyst promotes further reaction to the undesired bis- product species.

Bearing in mind the problems and deficiencies of the prior art, it is therefore an object of the present invention to provide a method of making hydroxyalkyl hindered phenolic antioxidants which uses low levels of metal catalyst and permits facile removal of most or all of the metal catalyst at the end of the reaction.

It is another object of the present invention to provide a method of making hydroxyalkyl hindered phenolic antioxidants wherein further reaction to the undesirable bis- product species is terminated or controlled.

Still other objects and advantages of the invention will in part be obvious and will in part be apparent from the specification.

### Summary of the Invention

The above and other objects and advantages, which will be apparent to one of skill in the art, are achieved in the present invention which is directed to, in a first aspect, a process of making a hindered phenolic antioxidant comprising:
providing an ester represented by 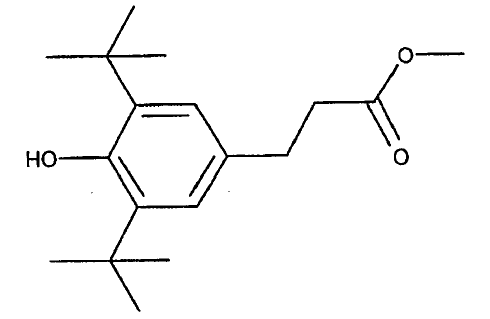 selected from the group consisting of ethylene glycol, 1,2-propane diol, 1,3-propane diol, 1,2-butane diol, 1,3-butane diol, 1,4-butane diol, 2,3-butane diol, 1,2-pentane diol, 1,3-pentane diol, 1,4-pentane diol, 1,5-pentane diol, 2,3-pentane diol, 2,4-pentane diol, 1,2-hexane diol, 1,3-hexane diol, 1,4-hexane diol, 1,5-hexane diol, 1,6-hexane diol, 2,3-hexane diol, 2,4-hexane diol, and 2,5-hexane diol. It is also useful to provide an excess of the diol. The process of this aspect may further include the step of removing any residual diol after the step of deactivating the metal catalyst.

Providing a metal catalyst selected from the group consisting of butyltin tris(2-ethylhexanoate), dibutyltin bis(2-ethylhexanoate), dibutyltin diacetate, dibutyltin oxide, trioctyltin oxide, butyltin trichloride, butyltin trimethylate, dibutyltin dichloride, diphenyltin dichloride, tributyltin chloride, stannous bis(2-ethylhexanoate), ethyl titanate, n-propyl titanate, titanium isopropoxide, n-butyl titanate polymer, 2-ethylhexyl titanate, cresyl titanate, titanium acetyl acetonate, titanium lactic acid chelate, and titanium triethanolaminato isopropoxide m an amount of 20 to 2000 ppm based on the amount of ester.

In the step of isolating the hindered hydroxyalkyl phenolic antioxidant, a resultant hindered hydroxyalkyl phenolic antioxidant has a residual metal level of less than 80 ppm, and preferably less than 10 ppm, based on an amount of the hindered hydroxyalkyl phenolic antioxidant.

Preferably, the step of deactivating the metal catalyst comprises deactivating the metal catalyst with an oxidizing agent, which may also be followed by treating the hindered hydroxyalkyl phenolic antioxidant with acid treated clay to further reduce a residual metal level of the antioxidant. The oxidizing agent is preferably selected from the group consisting of hydroperoxides, peroxides, manganese salts, oxygen, chlorine, perchlorates, and hypochlorites, wherein hydrogen peroxide is most preferred.

The step of deactivating the metal catalyst may also comprise deactivating the metal catalyst with a reducing agent. Upon deactivation, the residual metal level may be further reduced by treatment of the hindered hydroxyalkyl phenolic antioxidant with acid treated clay. The reducing agent is preferably selected from the group consisting of hydrazine or substituted hydrazines, sodium borohydride, lithium aluminum hydride, and borane complexes, wherein hydrazine or substituted hydrazines are most preferred.

As a one step process of deactivation and reduction of residual metal levels, one can use clay, acid treated clay, or acid treated bentonite clay. A residual metal content after the clay treatment is generally less than or equal to 6 ppm.

In another aspect, the present invention is directed to a method of making a hindered hydroxyalkyl phenolic antioxidant comprising the steps of:
providing an ester represented by methyl-β-(3,5-di-t-butyl-4-hydroxyphenyl) propionate;
providing, in excess, a diol selected from the group consisting of ethylene glycol, 1,2-propane diol, 1,3-propane diol, 1,2-butane diol, 1,3-butane diol, 1,4-butale diol, 2,3-butane diol, 1,2-pentane diol, 1,3-pentane diol, 1,4-pentane diol, 1,5-pentane diol, 2,3-pentane diol, 2,4-pentane diol, 1,2-hexane diol, 1,3-hexane diol, 1,4-hexane diol, 1,5-hexane diol, 1,6-hexiane diol, 2,3-hexane diol, 2,4-hexiane diol, and 2,5-hexane diol;
providing a tin or titanium catalyst in an amount of 20 to 500 ppm based on an amount of the ester;
heating the ester and the diol in the presence of the catalyst at a temperature of 150 to 220°C;
deactivating the catalyst;
treating the product mixture with clay and filtering; and
removing any residual diol after deactivation to isolate the product mixture, wherein the product mixture has a residual tin or titanium level of less than or equal to 6 ppm.

The tin catalyst is selected from the group consisting of butyltin tris (2-ethylhexanoate), dibutyltin bis(2-ethylhexanoate), dibutyltin diacetate, dibutyltin oxide, trioctyltin oxide, butyltin trichloride, butyltin trimethylate, dibutyltin dichloride, diphenyltin dichloride, tributyltin chloride, and stannous bis(2-ethylhexanoate). The titanium catalyst is selected from the group consisting of ethyl titanate, n-propyl titanate, titanium isopropoxide, n-butyl titanate polymer, 2-ethylhexyl titanate, cresyl titanate, titanium acetyl acetonate, titanium lactic acid chelate, and titanium triethanolaminato isopropoxide.

In yet another aspect, the present invention is directed to a method of making a hindered phenolic antioxidant comprising:
providing an ester represented by 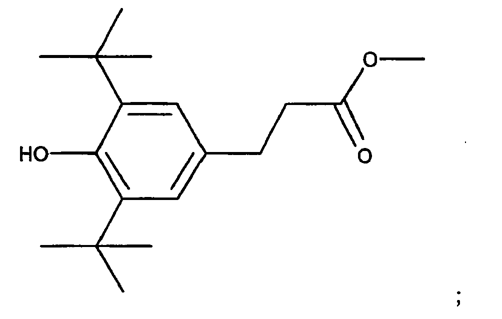
providing a diol represented by 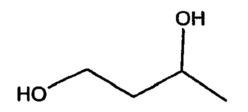
providing a tin or titanium catalyst in an amount of 20 to 500 ppm based on an amount of the ester;
heating the ester and the diol in the presence of the catalyst at a temperature of 180 to 205°C for a sufficient time to effectuate transesterification;
deactivating the catalyst with acid treated bentonite clay; and
isolating a reaction product having a residual tin or titanium content of less than or equal to 6 ppm.

Preferably, the step of providing a tin or titanium catalyst comprises providing a catalyst selected from the group consisting of butyltin tris(2-ethylhexanoate), dibutyltin bis(2-ethylhexanoate), dibutyltin diacetate, dibutyltin oxide, trioctyltin oxide, butyltin trichloride, butyltin trimethylate, dibutyltin dichloride, diphenyltin dichloride, tributyltin chloride, stannous bis(2-ethylhexanoate), ethyl titanate, n-propyl titanate, titanium isopropoxide, n-butyl titanate polymer, 2-ethylhexyl titanate, cresyl titanate, titanium acetyl acetonate, titanium lactic acid chelate, and titanium triethanolaminato isopropoxide.

Preferably, the hindered phenolic antioxidant has a residual metal content of equal to or less than 3 ppm.

The amount of diol is varied to control the amount of the desired product which is the hydroxyl-terminated mono-species. Low levels of the diol result in higher levels of the undesirable bis- species wherein the ester has added twice to the diol. A useful molar ratio of ester to diol is about 1:0.9 to about 1:5, preferably about 1:1 to about 1:4.

Many of the titanium catalysts are commercially available from E.I. DuPont DeNemours Company, Wilmington, Delaware, under the TYZOR^{®} tradename.

An effective amount of the catalyst is quite small, for tin, 10 to 200 ppm, preferably 20 to 100 ppm, and more preferably 20 to 60 ppm, and for titanium 50 to 3000 ppm, preferably 100 to 1000 ppm and more preferably 200 to 500 ppm, based on the amount of the ester. Using low levels of catalyst result in correspondingly low levels of residual metal in the final product of less than 3 to 80 ppm, preferably less than 30 ppm, and may be less than or equal to 20 ppm based on an amount of the final product. The residual metal level may be further reduced as discussed below.

By running the reaction with excess diol at temperatures of 150 to 220°C, the diol reacts to form primarily the desired hydroxyl-terminated mono- product species rather than the undesirable bis- product species. Furthermore, by deactivating the catalyst prior to removing the residual excess diol, further reaction to the bis-species is advantageously avoided. Subsequent removal of the residual diol by such means as vacuum stripping does not substantially alter the mono-/bis- product ratio once the catalyst is deactivated. Without deactivation of the catalyst prior to removal of the residual diol, however favorable the reaction is towards the desired mono- product species, further conversion to the undesirable bis- product species during work up of the reaction is highly likely.

Deactivating the metal catalyst may be accomplished by using small amounts of oxidizing agents, reducing agents, or clay treatment. Suitable oxidizing agents of even moderate activity may be used to deactivate the metal catalyst including, but not limited to, peroxides, hydroperoxides such as t-butyl hydroperoxide, manganese salts, oxygen, chlorine, perchlorates, and hypochlorites including sodium hypochlorite. A most preferred oxidizing agent is hydrogen peroxide. An effective amount of the oxidizing agent to deactivate the metal catalyst may be expressed as the oxidizing agent to catalyst molar ratio of 1:1 to 10:1, preferably 2:1.

Suitable reducing agents include, but are not limited to, hydrazine, substituted hydrazines such as methyl hydrazine, sodium borohydride, metal hydrides including lithium aluminum hydride, and borane complexes including borane-t-butylamine complex A most preferred reducing agent is hydrazine. An effective amount of the reducing agent to deactivate the metal catalyst may be expressed as the reducing agent to catalyst molar ratio of 1:1 to 10:1, preferably 2:1.

Clay, preferably acid treated clay, and most preferably acid treated bentonite clay, is an excellent means of deactivating the metal catalyst. It is understood that the term "bentonite" shall include all mineralogically related clays, such as smectites and montmorillonites, as set forth in the Kirk-Othmer Encyclopedia of Chemical Technology, 4th Edition, Vol. 6, pp. 381-405, the relevant portions of which are incorporated herein by reference. A preferred bentonite clay is commercially available as FILTROL™ from Engelhard Corporation of Jackson, Mississippi. Unexpectedly, when using the clay treatment, in particular acid treated day, not only is the metal catalyst deactivated but, upon filtration, the residual metal level is also significantly lower so that additional steps which may be needed to further lower residual metal levels are unnecessary. The clay is likely to absorb the metal catalyst into its pores thereby physically preventing the catalyst from promoting further reaction. Advantageously, the use of acid treated clay, and more preferably acid treated bentonite clay, in deactivating and absorbing the metal catalyst also reduces the residual metal level of the product mixture to less than 10 ppm based on an amount of the product mixture, preferably to less than or equal to 6 ppm, and most preferably to less than or equal to 3 ppm. Thus, using clay, preferably acid treated clay, provides a two-fold benefit in deactivating the metal catalyst and substantially reducing residual metal levels.

Upon deactivation of the metal catalyst, during isolation of the ester product, preferably by vacuum distillation, the ratio of mono- product species to bis- product species remains substantially constant. There is no live catalyst to generate further reactions with the desired mono- product species.

By deactivating the metal catalyst prior to isolating the final product, there is little change in the ratio between the desired mono-product species and the undesirable bis- product species since further reaction with the mono- product species cannot occur. The method of the present invention also provides unexpectedly low levels of residual tin or titanium in the final product mixture. The resultant product mixture can be used without further purification providing tremendous cost savings and efficiency in commercial processes, particularly when the acid treated bentonite clay is used to deactivate the metal catalyst. The resulting transesterification product may be used directly in subsequent synthesis steps or as an ingredient in mineral oils and formulated mineral oils with much improved solubility.

### Examples

The following Examples illustrate the preferred embodiments of the invention.

### Example 1 (Comparative)

Reaction of methyl (3,5-di-t-butyl-4-hydroxyphenyl) propionate and 1,2-propane diol.

This example illustrates the effect on the mono-/bis- product species ratio when the catalyst has not been deactivated prior to removing the excess diol.

In a 250mL 3-neck round bottom flask equipped with a Claisen adapter, an overhead stirrer and a short-path condenser, were combined 0.427 moles (125.0g) methyl (3, 5-di-t-butyl-4-hydroxyphenyl)propionate and 0.533 moles (40.6g) of 1,2-propane diol. On the Claisen adapter was a thermocouple and nitrogen inlet. A moderate nitrogen flow was maintained throughout the first part of the experiment. The reaction mixture was heated to 100°C and 0.68 mmoles (0.24g) of dibutyl tin diacetate was added. The system was then heated to 170°C. After 16 hours the level of starting propionate was below 1% and the reaction was terminated. The nitrogen was discontinued and vacuum applied. The vacuum (28.5 in. Hg) was maintained at a temperature of 170°C for 20 minutes to strip away residual diol. Analysis of the product by gas chromatography before sti-ipping showed 69.3 area% desired mono- species and 29.1 area% bis- species. After stripping the distribution changed to 35.4 area% mono- species and 63.9 area% bis- species.

### Example 2 (Catalyst Deactivation with Hydrazine)

Reaction of methyl (3,5-di-t-butyl-4-hydroxyphenyl) propionate and 1,3-butane diol.

This example illustrates the use of a reducing agent, hydrazine monohydrate, to deactivate the tin catalyst.

In a 250mL 3-neck round bottom flask equipped with a Claisen adapter, an overhead stirrer and a knock-back condenser (70-75°C) followed by a distillation condenser and receiver, were combined 0.257 moles (75.0g) methyl (3,5-di-t-butyl-4-hydroxyphenyl) propionate and 0.772 moles (69.6g) of 1,3-butane diol. On the Claisen adapter was a thermocouple and nitrogen inlet. A moderate nitrogen flow was maintained throughout the first part of the experiment. The mixture was heated to 100°C and 0.39 mmoles (0.14g) of dibutyl tin diacetate was added. The system was then heated to reflux conditions at 204°C. After 1.5 hours the level of starting propionate was below 1% and the reaction was terminated. The reaction was cooled to 50°C and 1.8 mmoles (0.09g) hydrazine monohydrate was added. After 1 hour of agitation, the system was heated to 120°C. The nitrogen was turned off and vacuum applied. The vacuum (28.5 in. Hg) was maintained at a temperature of 120°C for 30 minutes to strip away residual diol. Analysis of the product by gas chromatography before stripping showed 78.5 area% mono-species and 21.5 area% bis-species. After stripping the product distribution was 80.2 area% mono- species and 19.8 area% bis- species. Clearly, there is no undesirable change in the ratio of the mono-/bis- species after removal of residual diol once the catalyst is deactivated.

### Example 3 (Catalyst Deactivation with Hydrogen Peroxide)

Reaction of methyl (3,5-di-t-butyl-4-hydroxyphenyl) propionate and 1,3-butane diol.

This example illustrates the use of an oxidizing agent to deactivate the tin catalyst.

In a 2000mL 3-neck resin kettle reactor equipped with a Claisen adapter, an overhead stirrer and a knock-back condenser (70-75°C) followed by a distillation condenser and receiver, were combined 2.05 moles (600.0g) methyl (3,5-di-t-butyl-4-hydroxyphenyl) propionate and 6.16 moles (555.1g) of 1,3-butane diol. On the Claisen adapter was a thermocouple and nitrogen inlet. A moderate nitrogen flow was maintained throughout the first part of the experiment. The mixture was heated to 100°C and 0.57 mmoles (0.20g) of dibutyl tin diacetate was added. The system was then heated to reflux conditions at 204°C. After 4 hours the level of starting propionate was below 1% and the reaction was terminated. The reaction was cooled to 50°C and 1.1 mmoles (0.13g of a 30wt.% solution) hydrogen peroxide was added. After 1 hour of agitation, the system was heated to 120°C. The nitrogen was turned off and vacuum applied. The vacuum (28.5 in. Hg) was maintained at a temperature of 120°C for 1 hour to strip away residual diol. Analysis of the product by gas chromatography before stripping showed 82.3 area% mono- species and 16.0 area% bis- species. After stripping, the product distribution was 82.7 area% mono- species and 16.4 area% bis- species. Clearly, there is no undesirable change in the ratio of the mono-/bis- species after removal of residual diol once the catalyst is deactivated.

### Example 4 (Catalyst Deactivation with Hydrogen Peroxide & Residual Tin Removal With Acid Treated Bentonite Clay)

Reaction of methyl (3,5-di-t-butyl-4-hydroxyphenyl) propionate and 1,3-butane diol.

This example illustrates deactivation of the tin catalyst with an oxidizing agent and subsequent removal of residual tin with the acid treated bentonite clay to further lower the residual tin level.

In a 3000mL 3-neck round bottom flask equipped with a Claisen adapter, an overhead stirrer and a knock-back condenser (70-75°C) followed by a distillation condenser and receiver, were combined 4.28 moles (1250.0g) methyl (3,5-di-t-butyl-4-hydroxyphenyl) propionate and 12.83 moles (1157g) of 1,3-butane diol. On the Claisen adapter was a thermocouple and nitrogen inlet. A moderate nitrogen flow was maintained throughout the first part of the experiment. The mixture was heated to 100°C and 0.57 mmoles (0.19g) of dibutyl tin diacetate was added. The system was then heated to reflux conditions at 200-204°C. After 4.5 hours the level of starting propionate was below 1% and the reaction was terminated. The reaction was cooled to 50°C and 0.0013 moles (0.15g of a 30 wt.% solution) hydrogen peroxide was added. After 1 hour of agitation, the system was heated to 120°C. The nitrogen was turned off and vacuum applied. The vacuum (28.5 in Hg) was maintained for 30 minutes at 120°C to strip off the excess diol. The nitrogen flow was reestablished and the reaction cooled to 110°C to which was added 10.0g of FILTROL™ 20x acid treated bentonite clay and 10.0g of diatomaceous earth filter aid. The reaction was agitated for 30 minutes and then pressure filtered with a 1 micron filter disc at 100 psig nitrogen. Elemental analysis of the product before acid-clay treatment showed 19 ppm tin. Elemental analysis of the product after acid-clay treatment showed less than 3 ppm tin.

### Example 5 (Catalyst Deactivation & Residual Tin Removal with Acid Treated Bentonite Clay)

Reaction of methyl (3,5-di-t-butyl-4-hydroxyphenyl) propionate and 1,3-butane diol.

This example illustrates the use of acid treated bentonite clay for both deactivation of the tin catalyst and to lower residual tin levels.

In a 500mL 3-neck round bottom flask equipped with a Claisen adapter, an overhead stirrer and a knock-back condenser (70-75°C) followed by a distillation condenser and receiver, were combined 0.684 moles (200.0g) methyl (3,5-di-t-butyl-4-hydroxyphenyl) propionate and 2.053 moles (185.1g) of 1,3-butane diol. On the Claisen adapter was a thermocouple and nitrogen inlet. A moderate nitrogen flow was maintained throughout the first part of the experiment. The mixture was heated to 100°C and 0.15 mmoles (0.054g) of dibutyl tin diacetate was added. The system was then heated to reflux conditions at 200-207°C. After 5 hours the level of starting propionate was below 1% and the reaction was terminated. The reaction was cooled to 120°C and 1.7g of FILTROL™ 20x (acid treated bentonite clay) and 3.3g of diatomaceous earth filter aid were added. The reaction mass was filtered after 30 minutes of agitation. The residual diol was removed by vacuum distillation at 120°C for 1 hour. Analysis of the product by gas chromatography before stripping showed 87 area% mono- species and 13 area% bis- species. After stripping, the product distribution was 85 area% mono- species and 15 area% bis- species. Elemental analysis of the final product before acid clay treatment showed 60 ppm Sn. Analysis of the final product after acid clay treatment showed less than 5 ppm residual tin.

### Example 6 (Catalyst Deactivation and Residual Titanium Removal with Acid Treated Bentonite Clay)

Reaction of methyl (3,5-di-t-butyl-4-hydroxyphenyl) propionate and 1,3-butane diol with titanium (IV) (triethanolaminato) isopropoxide catalyst.

This example illustrates the use of acid treated bentonite clay for deactivation of the titanium catalyst and removal of residual titanium.

In a 250mL 3-neck round bottom flask equipped with a thermocouple adapter were combined 0.276 mol (80.45g) methyl (3,5-di-t-butyl-4-hydroxyphenyl) propionate and 1.14 mol (185.1g) of 1,3-butane diol. The flask was fitted with a thermocouple, a subsurface fritted glass nitrogen inlet, an overhead stirrer and a knock-back condenser (70-75°C) followed by a distillation condenser and receiver. A moderate nitrogen flow was maintained throughout the first part of the experiment. The mixture was heated to 82°C and 0.66 mmol (0.192g) of TYZOR^{®} TE titanium (IV) triethanolaminato isopropoxide (obtained from E. I. DuPont DeNemours Company) was added. The system was then heated at 190°C for 3.8 hours. When the level of starting propionate was below 1%, the reaction was terminated. The reaction was cooled to 120°C and 4.0g. FILTROL™ 20X (acid treated bentonite clay) was added. The reaction mass was stirred for 30 minutes and then filtered. The residual diol was removed by vacuum distillation at 132-140°C for 90 minutes. Analysis of the product by gas chromatography before clay treatment showed 93.0 area% mono-species and 6.7 area% bis-species. After stripping, the product distribution was 92.9 area % mono-species and 6.8 area % bis-species. Elemental analysis of the product before acid clay treatment showed 108 ppm titanium. Analysis of the filtered, clay treated product showed less than 3 ppm residual titanium.

### Example 7 (Catalyst Deactivation & Residual Titanium Removal with Acid Treated Bentonite Clay)

Reaction of methyl (3,5-di-t-butyl-4-hydroxyphenyl) propionate and 1,3-butane diol with titanium (IV) isopropoxide catalyst.

This example illustrates the use of acid treated bentonite clay for deactivation of the titanium catalyst and removal of residual titanium.

In a 250mL 3-neck round bottom flask equipped with a thermocouple adapter were combined 0.276mol (80.52g) methyl (3,5-di-t-butyl-4-hydroxyphenyl) propionate and 0.875mol (78.72g) of 1,3-butane diol. The flask was fitted with a thermocouple, a subsurface fritted glass nitrogen inlet, an overhead stirrer and a knock-back condenser (70-75°C) followed by a distillation condenser and receiver. A moderate nitrogen flow was maintained throughout the first part of the experiment. The mixture was heated to 120°C and 0.72mmol (0.212g) of TYZOR^{®} TPT titanium (IV) isopropoxide (obtained from E. I. DuPont DeNemours Company) was added. The system was then heated at 190°C for 4.0 hours. When the level of starting propionate was below 1%, the reaction was terminated. The reaction was cooled to 120°C and 4.0 g. FILTROL™ 20X (acid treated bentonite clay) was added. The reaction mass was stirred for 30 minutes and then filtered. The residual diol was removed by vacuum distillation at 130-140 °C for 40 minutes. Analysis of the product by gas chromatography before clay treatment showed 90.8 area % mono-species and 9.0 area % bis-species. After stripping, the product distribution was 90.4 area % mono-species and 9.4 area % bis- species. Elemental analysis of the product before acid clay treatment showed 190 ppm titanium. Analysis of the filtered, clay treated product showed less than 3 ppm residual titanium.

The present invention achieves the objects recited above. By deactivating the metal catalyst prior to removing any remaining diol in the transesterification reaction, the favorable mono-/bis- product species ratio is maintained. Deactivating the metal catalyst with acid treated bentonite clay provides a final product mixture with exceedingly low residual metal levels.

## Claims

1. A process of making a hindered phenolic antioxidant comprising:
a) providing an ester represented by 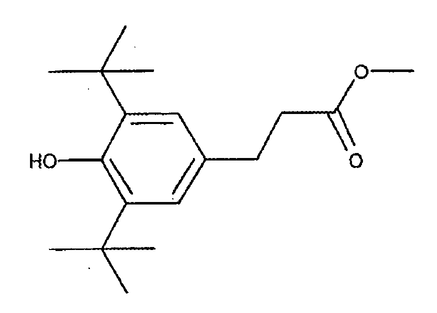
b) providing a diol selected from the group consisting of ethylene glycol, 1,2-propane diol, 1,3-propane diol, 1,2-butane diol, 1,3-butane diol, 1,4-butane diol, 2,3-butane diol, 1,2-pentane diol, 1,3-pentane diol, 1,4-pentane diol, 1,5-pentane diol, 2,3-pentane diol, 2,4-pentane diol, 1,2-hexane diol, 1,3-hexane diol, 1,4-hexane diol, 1,5-hexane diol, 1,6-hexane diol, 2,3-hexane diol, 2,4-hexane diol, and, 2,5-hexane diol;
c) providing a metal catalyst selected from the group consisting of butyltin tris(2-ethylhexanoate), dibutyltin bis(2-ethylhexanoate), dibutyltin diacetate, dibutyltin oxide, trioctyltin oxide, butyltin trichloride, butyltin trimethylate, dibutyltin dichloride, diphenyltin dichloride, tributyltin chloride, stannous bis(2-ethylhexanoate), ethyl titanate, n-propyl titanate, titanium isopropoxide, n-butyl titanate polymer, 2-ethylhexyl titanate, cresyl titanate, titanium acetyl acetonate, titanium lactic acid chelate, and titanium triethanolaminato isopropoxide;
d) heating the ester and the diol in the presence of the metal catalyst at a temperature of 150 to 220°C;
e) deactivating the metal catalyst; and
f) isolating the hindered phenolic antioxidant after deactivation.

2. The method of claim 1 wherein the step of providing the diol comprises providing an excess of the diol.

3. The method of claim 2 further including the step of removing any residual diol after the step of deactivating the metal catalyst.

4. The method of claim 1 wherein in the step of isolating the hindered phenolic antioxidant, a resultant hindered phenolic antioxidant has a residual metal level of less than 80 ppm based on an amount of the hindered phenolic antioxidant.

5. The method of claim 1 wherein the step of deactivating the metal catalyst comprises providing clay, acid treated clay, or acid treated bentonite clay.

6. The method of claim 1 wherein the step of deactivating the metal catalyst comprises deactivating the metal catalyst with an oxidizing agent or a reducing agent, whereby the oxidizing agent is selected from the group consisting of peroxides, hydroperoxides, manganese salts, oxygen, chlorine, perchlorates, and hypochlorites, and the reducing agent is selected from the group consisting of hydrazine or substituted hydrazines, sodium borohydride, metal hydrides, and borane complexes.

7. The method of claim 6 further including the step of treating the hindered phenolic antioxidant with clay, acid treated clay, or acid treated bentonite clay to further reduce a residual metal level of the antioxidant.

8. A method according to claim 1 of making a hindered phenolic antioxidant comprising the steps of:
providing an ester represented by methyl-β-(3,5-di-t-butyl-4-hydroxyphenyl)propionate;
providing, in excess, a diol selected from the group consisting of ethylene glycol, 1,2-propane diol, 1,3-propane diol, 1,2-butane diol, 1,3-butane diol, 1,4-butane diol, 2,3-butane diol, 1,2-pentane diol, 1,3-pentane diol, 1,4-pentane diol, 1,5-pentane diol, 2,3-pentane diol, 2,4-pentane diol, 1,2-hexane diol, 1,3-hexane diol, 1,4-hexane diol, 1,5-hexane diol, 1,6-hexane diol, 2,3-hexane diol, 2,4-hexane diol, and 2,5-hexane diol;
providing a tin or titanium catalyst in an amount of 20 to 500 ppm based on an amount of the ester;
heating the ester and the diol in the presence of the tin or titanium catalyst at a temperature of 150 to 220°C;
deactivating the tin or titanium catalyst;
treating the product mixture with clay and filtering
removing any residual diol after deactivation to isolate the product mixture, wherein the product mixture has a residual tin or titanium level of less than or equal to 6 ppm.

9. The method of claim 8 wherein the step of providing, in excess, a diol comprises providing 1,2-propane diol, 1,2-butane diol, 1,3-butane diol, 1,2-pentane diol, 1,4-pentane diol, 1,2-hexane diol or 1,5-hexane diol.

10. A method according to claim 1 of making a hindered phenolic antioxidant comprising:
a) providing an ester represented by 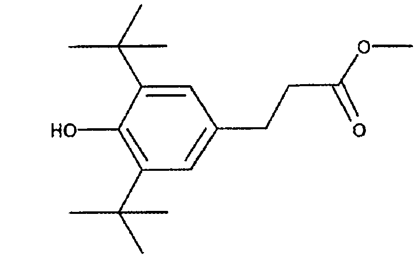
b) providing a diol represented by 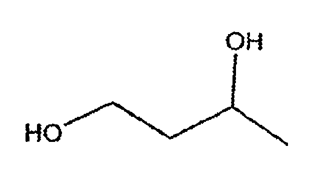
c)providing a tin or titanium catalyst in an amount of 20 to 500 ppm based on an amount of the ester;
d) heating the ester and the diol in the presence of the catalyst at a temperature of 180 to 205°C for a sufficient time to effectuate transesterification;
e) deactivating the catalyst with acid treated bentonite clay; and
f) isolating a reaction product having a residual tin or titanium content of less than or equal to 6 ppm.

11. The method of claim 10 wherein the tin or titanium catalyst is selected from the group consisting of butyltin tris(2-ethylhexanoate), dibutyltin bis(2-ethylhexanoate), dibutyltin diacetate, dibutyltin oxide, trioctyltin oxide, butyltin trichloride, butyltin trimethylate, dibutyltin dichloride, diphenyltin dichloride, tributyltin chloride, stannous bis(2-ethylhexanoate), ethyl titanate, n-propyl titanate, titanium isopropoxide, n-butyl titanate polymer, 2-ethylhexyl titanate, cresyl titanate, titanium acetyl acetonate, titanium lactic acid chelate, and titanium triethanolaminato isopropoxide.

12. The method of claim 1, wherein in the step of isolating the hindered phenolic antioxidant, a resultant hindered phenolic antioxidant has a residual metal level of less than 10 ppm based on an amount of the hindered phenolic antioxidant.

## Patentansprüche

1. Ein Verfahren zur Herstellung eines sterisch gehinderten phenolischen Antioxidans enthaltend:
a) Bereitstellung eines Esters repräsentiert durch 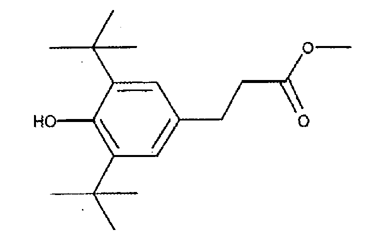
b) Bereitstellung eines Diols ausgewählt aus der Gruppe bestehend aus Ethylenglykol, 1,2-Propandiol, 1,3-Propandiol, 1,2-Butandiol, 1,3-Butandiol, 1,4-Butandiol, 2,3-Butandiol, 1,2-Pentandiol, 1,3-Pentandiol, 1,4-Pentandiol, 1,5-Pentandiol, 2,3-Pentandiol, 2,4-Pentandiol, 1,2-Hexandiol, 1,3-Hexandiol, 1,4-Hexandiol, 1,5-Hexandiol, 1,6-Hexandiol, 2,3-Hexandiol, 2,4-Hexandiol und 2,5-Hexandiol;
c) Bereitstellung eines Metallkatalysators ausgewählt aus der Gruppe bestehend aus Butylzinn-tris(2-ethylhexanoat), Dibutylzinn-bis(2-ethylhexanoat), Dibutylzinndiacetat, Dibutylzinnoxid, Trioctylzinnoxid, Butylzinntrichlorid, Butylzinntrimethylat, Dibutylzinndichlorid, Diphenylzinndichlorid, Tributylzinnchlorid, Zinn-bis(2-ethylhexanoat), Ethyltitanat, n-Propyltitanat, Titanisopropoxid, n-Butyltitanatpolymer, 2-Ethylhexyltitanat, Cresyltitanat, Titanacetylacetonat, Titan-Milchsäure-Chelat und Titantriethanolaminatisopropoxid;
d) Erhitzen des Esters und des Diols in Gegenwart des Metallkatalysators bei einer Temperatur von 150 bis 220°C;
e) Deaktivieren des Metallkatalysators und
f) Isolieren des sterisch gehinderten phenolischen Antioxidans nach Deaktivierung.

2. Die Methode des Anspruchs 1, worin der Schritt der Bereitstellung des Diols die Bereitstellung eines Überschusses des Diols enthält.

3. Die Methode des Anspruchs 2, weiterhin umfassend den Schritt der Entfernung jeglichen Restdiols nach dem Schritt der Deaktivierung des Metallkatalysators.

4. Die Methode des Anspruchs 1, worin in dem Schritt der Isolierung des sterisch gehinderten phenolischen Antioxidans ein resultierendes sterisch gehindertes phenolisches Antioxidans einen Restmetallgehalt von weniger als 80 ppm bezogen auf eine Menge des sterisch gehinderten phenolischen Antioxidans aufweist.

5. Die Methode des Anspruchs 1, worin der Schritt der Deaktivierung des Metallkatalysators die Bereitstellung von Tonerde, säurebehandelter Tonerde oder säurebehandelter Bentonit-Tonerde enthält.

6. Die Methode des Anspruchs 1, worin der Schritt der Deaktivierung des Metallkatalysators das Deaktivieren des Metallkatalysators mit einem Oxidationsmittel oder einem Reduktionsmittel enthält, wobei das Oxidationsmittel ausgewählt wird aus der Gruppe bestehend aus Peroxiden, Hydroperoxiden, Mangansalzen, Sauerstoff, Chlor, Perchloraten und Hypochloriten, und wobei das Reduktionsmittel ausgewählt wird aus der Gruppe bestehend aus Hydrazin oder substituierten Hydrazinen, Natriumborhydrid, Metallhydriden und Borankomplexen.

7. Die Methode des Anspruchs 6, weiterhin umfassend den Schritt der Behandlung des sterisch gehinderten phenolischen Antioxidans mit Tonerde, säurebehandelter Tonerde oder säurebehandelter Bentonit-Tonerde, um einen Restmetallgehalt des Antioxidans weiter zu reduzieren.

8. Eine Methode gemäß Anspruch 1 zur Herstellung eines sterisch gehinderten phenolischen Antioxidans enthaltend die Schritte:
Bereitstellung eines Esters repräsentiert durch Methyl-β-(3,5-di-t-butyl-4- hydroxyphenyl)propionat;
Bereitstellung eines Diols im Überschuss, ausgewählt aus der Gruppe bestehend aus Ethylenglykol, 1,2-Propandiol, 1,3-Propandiol, 1,2-Butandiol, 1,3-Butandiol, 1,4-Butandiol, 2,3-Butandiol, 1,2-Pentandiol, 1,3-Pentandiol, 1,4-Pentandiol, 1,5-Pentandiol, 2,3-Pentandiol, 2,4-Pentandiol, 1,2-Hexandiol, 1,3-Hexandiol, 1,4-Hexandiol, 1,5-Hexandiol, 1,6-Hexandiol, 2,3-Hexandiol, 2,4-Hexandiol und 2,5-Hexandiol;
Bereitstellung eines Zinn- oder Titankatalysators in einer Menge von 20 bis 500 ppm bezogen auf eine Menge des Esters;
Erhitzen des Esters und des Diols in Gegenwart des Zinn- oder Titankatalysators bei einer Temperatur von 150 bis 220 °C;
Deaktivieren des Zinn- oder Titankatalysators;
Behandeln der Produktmischung mit Tonerde und Filtrieren;
Entfernen jeglichen Restdiols nach Deaktivierung, um die Produktmischung zu isolieren, wobei die Produktmischung einen Restzinn- oder Resttitangehalt von weniger als oder gleich 6 ppm aufweist.

9. Die Methode des Anspruchs 8, worin der Schritt der Bereitstellung eines Diols im Überschuss das Bereitstellen von 1,2-Propandiol, 1,2-Butandiol, 1,3-Butandiol, 1,2-Pentandiol, 1,4-Pentandiol, 1,2-Hexandiol oder 1,5-Hexandiol enthält.

10. Eine Methode gemäß Anspruch 1 zur Herstellung eines sterisch gehinderten phenolischen Antioxidans enthaltend:
a) Bereitstellung eines Esters repräsentiert durch 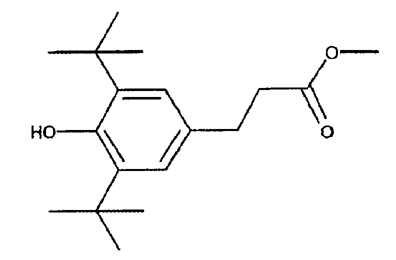
b) Bereitstellung eines Diols repräsentiert durch 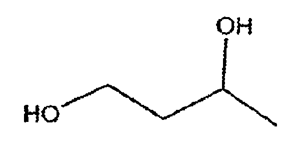
c) Bereitstellen eines Zinn- oder Titankatalysators in einer Menge von 20 bis 500 ppm bezogen auf eine Menge des Esters;
d) Erhitzen des Esters und des Diols in Gegenwart des Katalysators bei einer Temperatur von 180 bis 205 °C für eine ausreichende Zeit, um die Umesterung durchzuführen;
e) Deaktivieren des Katalysators mit säurebehandelter Bentonit-Tonerde und
f) Isolieren eines Reaktionsproduktes mit einem Restzinn- oder Resttitangehalt von weniger als oder gleich 6 ppm.

11. Die Methode des Anspruchs 10, worin der Zinn- oder Titankatalysator ausgewählt wird aus der Gruppe bestehend aus Butylzinn-tris(2-ethylhexanoat), Dibutylzinn-bis(2-ethylhexanoat), Dibutylzinndiacetat, Dibutylzinnoxid, Trioctylzinnoxid, Butylzinntrichlorid, Butylzinntrimethylat, Dibutylzinndichlorid, Diphenylzinndichlorid, Tributylzinnchlorid, Zinn-bis(2-ethylhexanoat), Ethyltitanat, n-Propyltitanat, Titanisopropoxid, n-Butyltitanatpolymer, 2-Ethylhexyltitanat, Cresyltitanat, Titanacetylacetonat, Titan-Milchsäure-Chelat und Titantriethanolaminatisopropoxid.

12. Die Methode des Anspruchs 1, worin in dem Schritt des Isolierens des sterisch gehinderten phenolischen Antioxidans ein resultierendes sterisch gehindertes phenolisches Antioxidans einen Restmetallgehalt von weniger als 10 ppm bezogen auf eine Menge des sterisch gehinderten phenolischen Antioxidans aufweist.

## Revendications

1. Procédé de fabrication d'un anti-oxydant phénolique encombré comprenant les opérations consistant à :
a) prendre un ester représenté par 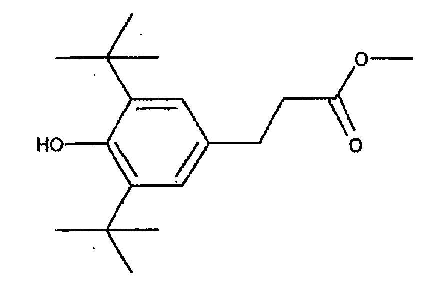
b) prendre un diol choisi dans le groupe constitué par l'éthylène glycol, le 1,2-propane diol, le 1,3-propane diol, le 1,2-butane diol, le 1,3-butane diol, le 1,4-butane diol, le 2,3-butane diol, le 1,2-pentane diol, le 1,3-pentane diol, le 1,4-pentane diol, le 1,5-pentane diol, le 2,3-pentane diol, le 2,4-pentane diol, le 1,2-hexane diol, le 1,3-hexane diol, le 1,4-hexane diol, le 1,5-hexane diol, le 1,6-hexane diol, le 2,3-hexane diol, le 2,4-hexane diol et le 2,5-hexane diol ;
c) prendre un catalyseur métallique choisi dans le groupe constitué par le tris(2-éthylhexanoate) de butylétain, le bis(2-éthylhexanoate) de dibutylétain, le diacétate de dibutylétain, l'oxyde de dibutylétain, l'oxyde de trioctylétain, le trichlorure de butylétain, le triméthylate de butylétain, le dichlorure de dibutylétain, le dichlorure de diphénylétain, le chlorure de tributylétain, le bis(2-éthylhexanoate) stanneux, le titanate d'éthyle, le titanate de n-propyle, l'isopropylate de titane, le titanate de n-butyle polymère, le titanate de 2-éthylhexyle, le titanate de crésyle, l'acétyl acétonate de titane, le chélate de titane acide lactique et le triéthanolaminato isopropylate de titane ;
d) chauffer l'ester et le diol en présence du catalyseur métallique à une température de 150 à 220 °C ;
e) désactiver le catalyseur métallique ; et
f) isoler l'anti-oxydant phénolique encombré après désactivation.

2. Procédé selon la revendication 1, dans lequel l'étape consistant à prendre le diol comprend prendre un excès du diol.

3. Procédé selon la revendication 2, comprenant en outre l'étape d'élimination de tout diol résiduel après l'étape de désactivation du catalyseur métallique.

4. Procédé selon la revendication 1, dans lequel, dans l'étape d'isolement de l'anti-oxydant phénolique encombré, un anti-oxydant phénolique encombré résultant a un taux de métal résiduel de moins de 80 ppm sur la base d'une quantité de l'anti-oxydant phénolique encombré.

5. Procédé selon la revendication 1, dans lequel l'étape de désactivation du catalyseur métallique comprend l'opération consistant à prendre de l'argile, de l'argile traitée par un acide ou de l'argile bentonite traitée par un acide.

6. Procédé selon la revendication 1, dans lequel l'étape de désactivation du catalyseur métallique comprend la désactivation du catalyseur métallique par un agent oxydant ou un agent réducteur, ce par quoi l'agent oxydant est choisi dans le groupe constitué par les peroxydes, les hydroperoxydes, les sels de manganèse, l'oxygène, le chlore, les perchlorates et les hypochlorites, et l'agent réducteur est choisi dans le groupe constitué par l'hydrazine ou les hydrazines substitués, le borohydrure de sodium, les hydrures métalliques et les complexes du borane.

7. Procédé selon la revendication 6, comprenant en outre l'étape consistant à traiter l'anti-oxydant phénolique encombré par de l'argile, de l'argile traitée par un acide ou de l'argile bentonite traitée par un acide pour réduire encore un taux de métal résiduel de l'anti-oxydant.

8. Procédé selon la revendication 1 de fabrication d'un anti-oxydant phénolique encombré comprenant les étapes consistant à :
- prendre un ester représenté par le β-(3,5-di-t-butyl-4-hydroxyphényl)propionate de méthyle ;
- prendre, en excès, un diol choisi dans le groupe constitué par l'éthylène glycol, le 1,2-propane diol, le 1,3-propane diol, le 1,2-butane diol, le 1,3-butane diol, le 1, 4-butane diol, le 2, 3-butane diol, le 1,2-pentane diol, le 1,3-pentane diol, le 1,4-pentane diol, le 1,5-pentane diol, le 2,3-pentane diol, le 2,4-pentane diol, le 1,2-hexane diol le 1,3-hexane diol, le 1,4-hexane diol, le 1,5-hexane diol, le 1,6-hexane diol, le 2,3-hexane diol, le 2,4-hexane diol, le 2,5-hexane diol ;
- prendre un catalyseur à l'étain ou au titane dans une quantité de 20 à 500 ppm sur la base d'une quantité de l'ester ;
- chauffer l'ester et le diol en présence du catalyseur à l'étain ou au titane à une température de 150 à 220 °C ;
- désactiver le catalyseur à l'étain ou au titane ;
- traiter le mélange obtenu comme produit par de l'argile et filtrer ;
- retirer tout diol résiduel après désactivation pour isoler le mélange obtenu comme produit, dans lequel le mélange obtenu comme produit a un taux résiduel d'étain ou de titane de moins de ou égal à 6 ppm.

9. Procédé selon la revendication 8, dans lequel l'étape consistant à prendre, en excès, un diol comprend l'opération consistant à prendre du 1,2-propane diol, du 1,2-butane diol, du 1,3-butane diol, du 1,2-pentane diol, du 1,4-pentane diol, du 1,2-hexane diol ou du 1,5-hexane diol.

10. Procédé selon la revendication 1 de fabrication d'un anti-oxydant phénolique encombré comprenant les opérations consistant à :
a) prendre un ester représenté par 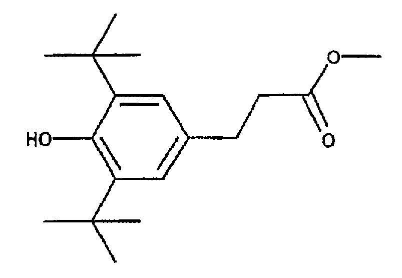
b) prendre un diol représenté par 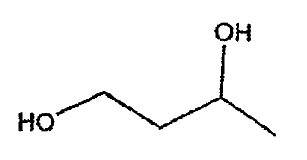
c) prendre un catalyseur à l'étain ou au titane dans une quantité de 20 à 500 ppm sur la base d'une quantité de l'ester ;
d) chauffer l'ester et le diol en présence du catalyseur à une température de 180 à 205 °C pendant un laps de temps suffisant pour effectuer une transestérification ;
e) désactiver le catalyseur par de l'argile bentonite traitée par un acide ; et
f) isoler un produit de réaction ayant une teneur résiduelle en étain ou titane de moins de ou égale à 6 ppm.

11. Procédé selon la revendication 10, dans lequel le catalyseur à l'étain ou au titane est choisi dans le groupe constitué par le tris(2-éthylhexanoate) de butylétain, le bis(2-éthylhexanoate) de dibutylétain, le diacétate de dibutylétain, l'oxyde de dibutylétain, l'oxyde de trioctylétain, le trichlorure de butylétain, le triméthylate de butylétain, le dichlorure de dibutylétain, le dichlorure de diphénylétain, le chlorure de tributylétain, le bis(2-éthylhexanoate) stanneux, le titanate d'éthyle, le titanate de n-propyle, l'isopropylate de titane, le titanate de n-butyle polymère, le titanate de 2-éthylhexyle, le titanate de crésyle, l'acétyl acétonate de titane, le chélate de titane acide lactique et le triéthanolaminato isopropylate de titane.

12. Procédé selon la revendication 1, dans lequel, dans l'étape d'isolement de l'anti-oxydant phénolique encombré, un anti-oxydant phénolique encombré résultant a un taux de métal résiduel de moins de 10 ppm sur la base d'une quantité de l'anti-oxydant phénolique encombré.
